# EUROPEAN PATENT APPLICATION

(11) **EP 1 114 618 A2**
(43) Date of publication of application: **11.07.2001**
(21) Application number: 01300022.9
(22) Date of filing: 03.01.2001
(51) Int. Cl.: A61B 17/064, A61F 2/02

(54) **Surgical instrument for applying beads to tissue**

(30) Priority: 04.01.2000 US 477747
(71) Applicant: ETHICON ENDO-SURGERY, INC., Cincinnati, OH 45242-2839 (US)
(72) Inventor: Bakos, Gregory J., Mason, Ohio 45040 (US); Daugird, Joanne, Cincinnati, Ohio 45236 (US); Thompson, Suzanne E., West Chester, Ohio 45069 (US)
(74) Representative: Mercer, Christopher Paul

(57) **Abstract**

A surgical instrument for the placement of a plurality of beads within wall tissue to create, or augment a sphincter within a lumen is disclosed. The surgical instrument has a hollow elongated flexible sheath having the plurality of beads stored within a passageway within the sheath. A tip is located at a distal end of the sheath. The tip is adapted for penetrating tissue and for expelling at least one of the plurality of beads therefrom. A feeding member extends into the sheath passageway of the sheath and is operably coupled to the plurality of beads. Moving the feeding member expels at least one of the plurality of beads from the distal tip. A tissue anchor is located at the tip of the elongated flexible sheath and the tissue anchor is actuatable to engage with the tissue wall. The tissue anchor restrains the tip relative to a selected surgical site for the placement of at least one of the plurality of beads therein. The tissue anchor is de-actuatable to disengage the tissue anchor from the tissue wall.

## Description

### Field of the Invention

The present invention relates, in general, to a surgical instrument and method for the placement of a plurality of beads within tissue of a patient and, more particularly, to a surgical instrument and method for the placement of a plurality of beads within tissue surrounding a lumen to augment or create a sphincter in the tissue.

### Background of the Invention

Gastro-Esophageal Reflux Disease ("GERD") is a disease state wherein the lower esophageal sphincter fails to close properly and gastric secretions or reflux migrate upwardly from the stomach to the lower portions of the esophagus. This condition causes heartburn or esophagitus in the patient. The occasional exposure of the esophagus to gastric secretions is not harmful, but chronic exposure can irritate the mucosal lining and create abnormal mucosal cells. In a certain percentage of the population, the abnormal cells can be a precursor to the development of esophageal cancer. Esophageal cancer is one of the most lethal of all cancers and initial diagnosis is difficult without a visual inspection of the esophagus.

Treatment of GERD ranges from the administration of antiacids in mild cases to complex surgery such as a Nissen fundoplication. The Nissen fundoplication requires surgical opening of the patient, and the invagination and suturing of a portion of the stomach around the lower portion of the esophagus to create an artificial esophageal sphincter. In some surgeries, a caustic is applied between the wrapped portion of the stomach and esophagus to induce scarring and to promote the growing together of the wrapped tissue. Due to the location of the esophagus within the thoracic cavity and its close proximity to the lungs, heart and other vascular structures, open surgery is a major undertaking. Due to age, health, severity of GERD, and other factors, not all patients are candidates for surgery such as the Nissen fundoplication. As a consequence, the medical profession has tended to treat GERD symptoms rather than eradicating the root cause.

When a patient is diagnosed as having GERD, the traditional treatment has been oral medication and lifestyle modifications and, as a last resort, surgical intervention such as a Nissen fundoplication. The medical community began looking for a minimally invasive method of repairing or replacing the lower esophageal sphincter that did not involve major surgery. U.S. Patent 5,571,116 by Bolanos et al. teach a minimally invasive stapling instrument that is inserted transorally to invaginate or fold the gastroesophageal junction of the stomach. The folded invaginated tissue is fastened together with staples to support or bolster the lower esophageal sphincter. U.S. Patent No. 5,887,594 by LoCicero, III teaches an alternate surgical device and alternate tissue fasteners to accomplish substantially the same task as Bolanos et al.

Whereas Bolanos et al. and the LoCicero, III did indeed address the need for a minimally invasive method of repairing the lower esophageal sphincter, they used a method that was a transoral adaption of the Nissen Fundoplication surgical procedure. Like the Nissen fundoplication procedure, muscular tissue was invaginated and fastened. Whereas the results were effective, the surgery was complicated and still had some of the trauma and longer patient recovery times associated with the Nissen fundoplication procedure.

What is needed was a minimally invasive surgical method of augmenting and repairing the lower esophageal sphincter that did not require the physical folding and stapling of muscular tissue, and reduced the long recovery times associated with this procedure. Augmentation of the sphincter is another approach to the problem of a sphincter muscle that cannot adequately close. By mucosally implanting a bulking material within soft tissue near the sphincter, the surgeon is able to reduce or close the damaged orifice by augmenting or swelling the sphincter defect. It is important that the placed bulking materials maintain their position and their shape to remain effective and that the bulking materials are biocompatible. Ideally, the physical properties (such as resiliency) of the bulking material should match that of the tissue into which it is placed, and as a consequence, many of the bulking materials contain resilient solids or semi-solid particles (or fibrils).

Bulking materials are best known in the art for their use in reconstructive surgery such as the treatment of cosmetic defects such as wrinkles, and for the augmentation of sphincters such as the urinary, anal, or gastroesophageal sphincter. The majority of the bulking material patents are written around the use of bulking materials for the treatment of urinary incontinence. The method of placement most generally used for the placement of bulking materials is injection from the needle of a syringe. The use of a needle and a syringe as an application device places some limitations on the bulking material. First, the particulates of the bulking material should be small enough to fit through the needle of the applicator, and second, the bulking material should be fluid enough to flow through the needle without requiring Herculean effort for expulsion of the bulking material through the needle.

Solid or semi-solid particles (or fibrils) that are small enough to fit through the needle of the applicator are sometimes referred to as micro particles. Micro particles can range in size from 1-3000 microns. A carrier medium or fluid is frequently mixed with the micro particles of the bulking material to make the bulking material fluid enough to carry the micro particles or fibrils from the syringe and into the tissue. The carrier medium holds the micro particles in suspension and acts as a lubricant during the expulsion of the micro particles from the needle. U.S. Patent No. 5,258,028 was issued to Erseck et al. wherein he teaches a preferred average range of sizes for the micro particles and the use of a carrier fluid. Additional patents describing limits on the micro particle size ranges and the use of a carrier fluid are U.S. Patent Nos. 5,258,028 by Ersk et al., U.S. Patent Nos. 5,451,406 and 5,792,478 by Lawin et al., PCT Application WO 98/01088 by Lawin et al. and published 1/15/98, PCT Application WO 98/44965 by David Healey and published 10/15/98, and PCT Application WO 99/31167 by Vanderhoff et al. and published 6/24/99. The Lawin et al. '478 patent and the Lawin et al. PCT Application WO 98/01088 specifically describe the use of bulking materials for the repair of the lower gastroesophageal sphincter.

There are several known problems with using micro particles or fibrils and with using a carrier medium. The size of the particle is important as small particles migrate from the application site to another area of the body, and large particles cannot fit down the needle of the syringe. The small particles are carried away by the capillary system and travel until they would reach a site within another organ. Large particles are not suitable for use with a syringe, as they cannot pass through the restriction of the needle orifice. Lubricious carrier mediums can exacerbate the migration of particles from the application site by making it easier for the particles to slip through local tissue restrictions. Additionally, the carrier medium itself is frequently a biocompatible material such as but not limited to saline, beta-glucan, natural oils, collagens, polyvinyl pyrrolidone or hydrogel, glycerol and others. Over time, the body absorbs some, or all, of the biocompatible carrier medium. Migration of the carrier medium can also occur when materials such as finely ground polyvinyl pyrrolidone (PVP or hydrogel) are used as a lubricant in the carrier medium. This absorption and/or migration shrinks the size of the tissue augmentation by shrinking the volume of the injected bulking material, and in some cases, necessitating additional treatment.

The migration of bulking materials can be avoided by using a balloon like containment membrane that acts as an envelope to retain the fluids and particulates within. The containment membrane is placed at a desired surgical site and is expanded by the injection of a bulking material having particulates and fluids or just a fluid. Once the bulking material expands the membrane, it is sealed. Haber et al. in U.S. Patents 4,773393 and 4,832,680 teaches syringe like instruments for the deployment of the particle containment membrane and bulking material. Haber does not disclose the use of his inventions for repair of the lower gastroesophageal sphincter. Additionally, the bulking provided by his inventions limited augmentation to a spherical shape placed in one localized area. The localized treatment described by Haber et al. was not conducive to augmentation of the ringlike lower esophageal sphincter.

The problem of the shrinking volume of the bulking material caused by the absorption of the carrier medium is avoided by eliminating the absorbable carrier medium, and using a pre-swollen particle material that is naturally lubricious when wet, such as swollen polyvinyl pyrrolidone (hydrogel). The lack of a carrier medium reduces the potential for shrinkage of the bulking material over time. The polyvinyl pyrrolidone (hydrogel) particles are pre-swollen by immersing them in a low molecular weight water-soluble organic compound, and drained. Van Bladel et al. in U.S. Patent No. 5,813,411 teaches placing the drained lubricious micro particles into a syringe and injecting them directly into the patient for the treatment of urinary disorders. Van Bladel et al. does not address the potential for the migration of the lubricious particles from the injection site. Eric Berg in U. S. Patent No. 5,007,940 addresses the migration of pre-swelled polyvinyl pyrrolidone (hydrogel) particles from the injection site by enlarging the size of the particles. Berg teaches an enlarged macro particle that ranges from .005 inches to .20 inches in diameter that can be passed through a needle of a syringe. The macro particle of Berg is larger than the internal diameter of the needle. To accomplish the passage of the large macro particle through the smaller needle orifice, the macro particles must be pre-swelled, have a lubricious surface, and capable of undergoing a deformation of about 20% to 80% as they travel down the undersized needle. However, Van Bladel et al. teaches the Berg macro particles are costly, require large diameter needles, and require relatively large insertion forces to pass the oversized macro particles through the undersized needle.

Schindler et al. teaches a soft tissue augmentation apparatus for use in soft tissue procedures (such as facial contouring procedures) in U.S. Patent No. 5,607,477. Schindler et al. shows two elongated pointed instruments that receive a flexible tubular implant around the shaft of the instrument. The elongated pointed instrument and attached flexible tubular implant are placed into tissue. When the instrument is retracted from tissue, the flexible tubular implant is left within the tissue. Schindler et al. does not teach the use of the soft tissue augmentation apparatus for use on the esophagus nor are the elongated tubular implants suitable for augmentation of the circular cross section of the lower esophageal sphincter.

Fujioka et al. in U. S. Patent No. 4,950,234 teaches an instrument for administering preparations from a needle of a syringe device. The Fujioka et al. differs from previous syringe devices in that the ejectable preparations are solid or semi-solid in composition and use no lubricants. The syringe device is similar in design to previous syringe bulking instruments and ejects the solid or semi- solid preparations from the needle of the syringe by moving a plunger. The particles or active ingredients described by Fujioka et al. are frequently a drug mixed with a carrier such as collagen, gelatin, albumin, and others. Fujioka et al. does not teach the use of the solid or semi- solid bulking materials for bulking tissue and does not teach the use of the above instrument on the esophagus.

It is important to note that the known devices or methods of augmenting or creating a sphincter are radical surgery, minimally invasive mechanical fastening devices, or the use of a bulking material. Presently, there are no known devices or methods that are minimally invasive, provide rapid patient recovery, and avoid the issues found with conventional bulking materials and instruments in the repair or augmentation of a sphincter, in particular, the lower gastroesophageal sphincter.

### Summary of the Invention

The present invention is a surgical instrument for the placement of a plurality of beads within a tissue wall surrounding a lumen in a patient, the plurality of beads creating or augmenting a sphincter therein.

The surgical instrument includes an elongated flexible sheath having a proximal, and a distal end, and a sheath passageway extending therebetween. The sheath passageway has the plurality of beads stored therein.

A distal tip is located at the distal end of the elongated flexible sheath. The distal tip is adapted for penetrating tissue and the distal tip has at least one opening therein for the expulsion of at least one of the plurality of beads therefrom.

A feeding member extends into the sheath passageway of the elongated flexible sheath and is operably coupled to the plurality of beads. The feeding member is moveable for the expulsion of at least one of the plurality of beads from the distal tip.

A tissue anchor is located at the distal tip of the elongated flexible sheath. The tissue anchor is actuatable to engage with the tissue wall to restrain the distal tip relative to a selected surgical site for the placement of at least one of the plurality of beads therein. The tissue anchor is de-actuatable to disengage the tissue anchor from the tissue wall.

Significantly, the novel surgical instrument for the placement of beads within tissue wall surrounding a lumen addresses a long felt need by surgeons. Specifically, the surgical instrument of the present invention avoids the need for radical surgery, and eliminates the need for surgical fastening instruments and their associative long recovery times. Consequently, in the preferred embodiment of this invention, the surgeon is provided with an improved surgical instrument for the repair of the lower gastroesophageal sphincter that is minimally invasive, promotes rapid patient recovery, and is adapted for use with a novel bead bulking material that reduces the need for additional surgery to re-augment the sphincter.

In a particularly preferred embodiment of the invention, the distal tip located at the distal end of the flexible sheath of the instrument of the present invention is tapered to penetrate tissue and has at least two flexible petals that are deflected outwardly for the passage of at least one of the beads from the tip and into the tissue wall. The flexible petals are preferably biased outwardly by the passage of the bead and return to a normally closed position. Thus the surgeon is provided with a tapered tissue-penetrating tip that can pass a large bead therefrom.

In another particularly preferred embodiment of the invention, the surgical instrument has an indexing mechanism operably coupled to the plurality of beads such that actuation of the indexing mechanism ejects a preferred number of the plurality of beads from the tip and into the tissue wall. Significantly, the indexing mechanism provides the surgeon with the ability to regulate the amount of beads that are used to augment the tissue wall.

In an alternate preferred embodiment of the present invention, the surgical instrument includes an elongated flexible overtube having a proximal end and a distal tapered end. The elongated flexible overtube has at least one tissue receiving port for the reception of a tissue portion therein. The at least one tissue receiving port is adjacent to the tapered end and communicates with a first overtube passageway that extends from the tissue receiving port to the proximal end of the elongated flexible overtube.

An elongated flexible sheath of the alternate embodiment is movably located within the first overtube passageway. The elongated flexible sheath has a proximal end, a distal end, and a sheath passageway extending therebetween. The sheath passageway has a plurality of beads stored therein.

A tip of the alternate embodiment is at the distal end of the elongated flexible sheath. The tip is for penetrating the tissue portion drawn within the tissue receiving port of the elongated flexible overtube. The tip has at least one opening therein for the expulsion of at least one of the plurality of beads therefrom.

A feeding member of the alternate embodiment extends into the sheath passageway of the elongated flexible sheath and is operably coupled to the plurality of beads. The feeding member is moveable for the expulsion of at least one of the plurality of beads from the tip of the flexible sheath.

A tissue anchor of the alternate embodiment is located at the tip of the elongated flexible sheath. The tissue anchor is actuatable to engage with the tissue portion within the tissue receiving port of the elongated flexible overtube. The tissue anchor restrains the tip relative to the tissue portion for the placement of at least one of the plurality of beads therein. The tissue anchor is de-actuatable to disengage the tissue anchor from the tissue wall.

The surgical instrument of this invention can be used in any surgical or medical procedure where it is necessary or desired to augment or to create a sphincter within a lumen. For a better understanding of the invention and its unique features, reference is made to the accompanying drawings and descriptive matter in which the preferred invention is fully described.

### Brief Description of the Drawings

The novel features of the invention are set forth with particularity in the appended claims. The invention itself, however, both as to organization and methods of operation, together with further objects and advantages thereof, may best be understood by reference to the following description, taken in conjunction with the accompanying drawings in which:
FIG. 1 is an isometric view of a surgical instrument of the present invention wherein a flexible sheath of the surgical instrument is placed into an operative channel of an endoscope;
FIG. 2 is an exploded isometric view of the internal elements of the surgical instrument of FIG. 1 with some elements sectioned for clarity;
   FIG. 3 is an isometric view of the assembled internal components of a handle portion of the surgical instrument of FIG. 1, with a right cover removed for clarity, and showing a proximal portion of a tissue anchor in the extended position, and an indexing mechanism ready to fire a bead;
FIG. 4 is an isometric view of the assembled internal components of a handle portion of the surgical instrument of FIG.3, with the tissue anchor in the retracted position and the indexing mechanism ready to expel a second bead.
FIG. 5 is a side view, in cross section, showing the elements of a distal portion of the flexible sheath of FIG. 1 wherein a distal portion of the tissue anchor is shown extended from a tip of the flexible sheath;
FIG. 6 is the side view of FIG. 5, wherein the distal portion of the tissue anchor is retracted into the tip of the flexible sheath, and one bead has been partially expelled from the tip of the flexible sheath;
Fig. 7 is the side view of FIG. 6, wherein one bead is shown fully expelled from the tip of the flexible sheath;
FIG. 8 is an isometric view of an overtube instrument for use in combination with the surgical instrument depicted in FIG. 1;
FIG. 9 is a cross sectional view of a patient wherein the endoscope has been inserted into the patient's mouth and esophagus to place the flexible sheath and tip of the surgical instrument of FIG. 1 near the lower esophageal sphincter;
FIG. 10 is a cross sectional view of the lower portion of the esophagus and the upper portion of the stomach showing the engagement of the tissue anchor with the tissue wall of the esophagus to restrain the tip relative to a selected surgical site;
FIG. 11 is the cross sectional view of FIG. 10 showing the advancement of the tip into a tissue wall of the esophagus and the placement of a dehydrated bead into a tissue pocket formed between an inner lining and a surrounding muscle layer of the esophagus;
FIG. 12 is the cross sectional view of FIG. 11 showing a second dehydrated bead expelled from the tip into a second tissue pocket formed between an inner lining and a surrounding muscle layer of the esophagus, the dehydrated beads augmenting the lower esophageal sphincter;
FIG. 13 is the cross sectional view of FIG.12 approximately one week after the surgery showing the expansion of the moisture absorbing beads by hydration from bodily fluids, the hydrated beads augmenting the lower esophageal sphincter;
FIG. 14 is a cross sectional view of the lower portion of the lumen or esophagus and showing the placement of the overtube instrument of FIG. 9 at a selected surgical site within the esophagus of a patient;
FIG. 15 is the cross sectional view of FIG. 14 showing the reception of a portion of the tissue wall into a tissue receiving port of the overtube instrument;
FIG. 16 is the cross sectional view of FIG. 15 showing the extension of the tissue anchor into the portion of the tissue wall received in the tissue receiving port;
FIG. 17 is the cross sectional view of FIG. 16 showing the advancement of the tip into a tissue wall of the esophagus and the placement of a moisture absorbing bead into a tissue pocket formed between an inner lining and a surrounding muscle layer of the esophagus.

### Detailed Description of the Invention

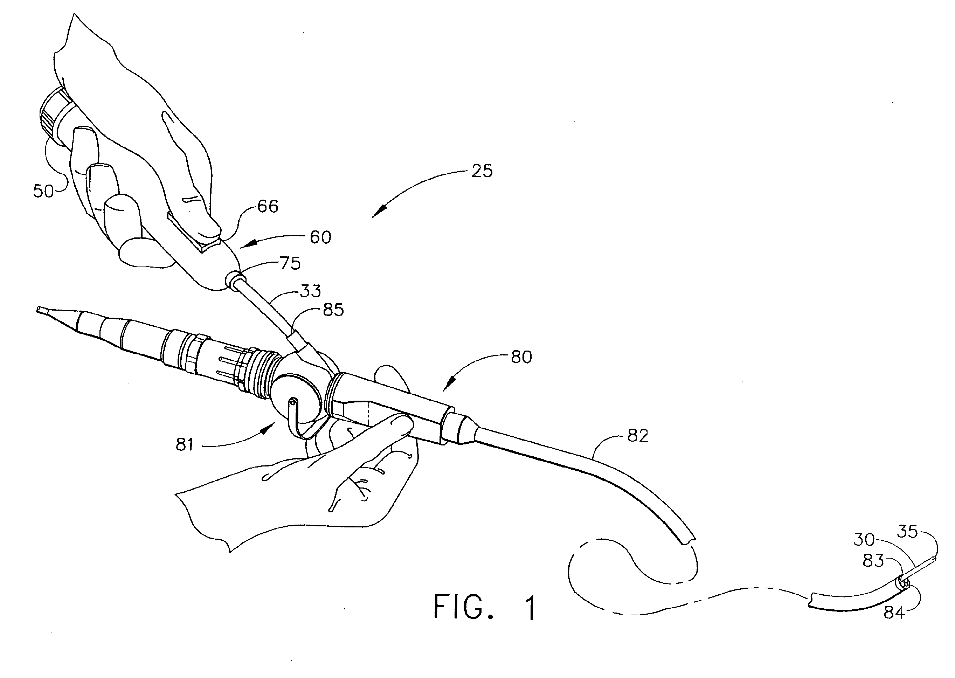

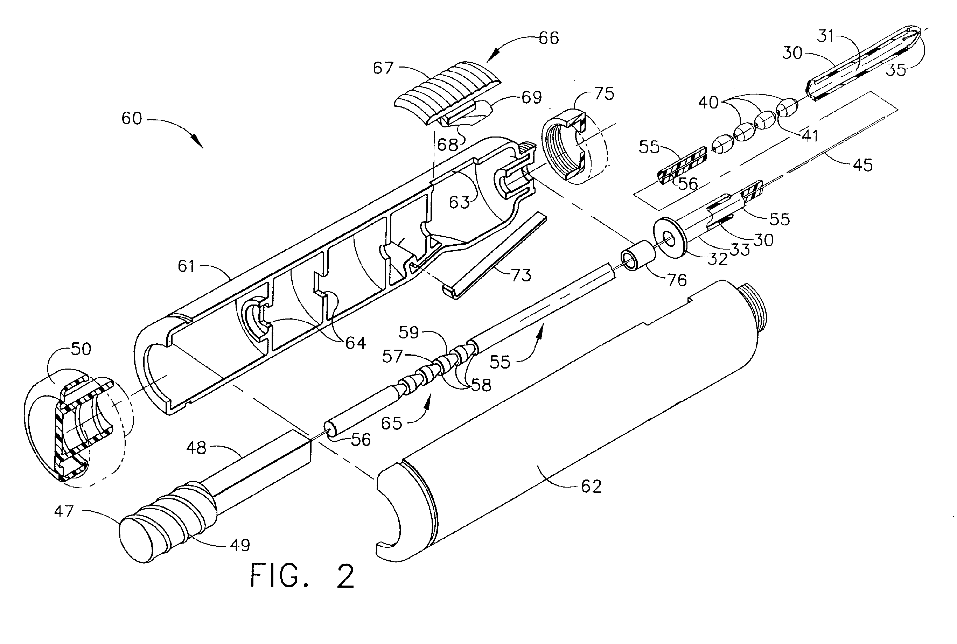

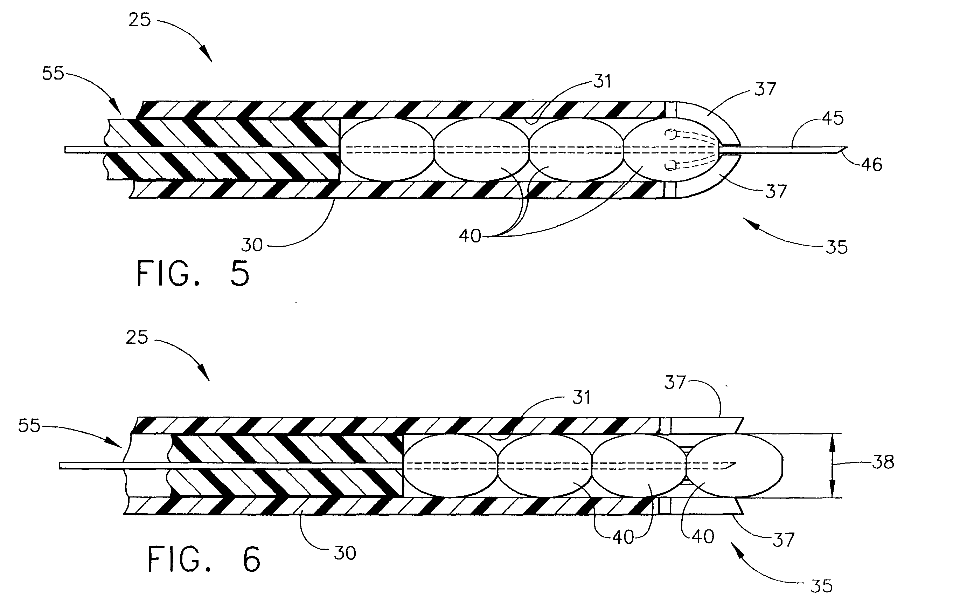

The present invention relates, in general, to a surgical instrument and method for the placement of a plurality of beads within tissue of a patient. More particularly, to a surgical instrument and method for the placement of a plurality of beads within tissue surrounding a lumen to augment or create a sphincter in the tissue. As shown in FIG. 1, the surgical instrument 25 of the present invention has an elongated flexible sheath 30, and a plurality of beads 40 stored within (FIG. 2). The flexible sheath 30 has a tip 35 at a distal end for piercing tissue, and an opening 38 (FIG. 6) within the tip 35 for the expulsion of beads 40 into tissue. A cylindrical strain relief 33 is located at a proximal end of the flexible sheath 30 and is of a larger diameter than the flexible sheath 30. A handle 60 is located at a proximal end of the surgical instrument 25 for the surgeon to grip. An indexing mechanism 65 (FIG. 2) is provided and operably couples a firing trigger 66 of the indexing mechanism 65 to the beads 40. Actuation of the firing trigger 66 actuates the indexing mechanism 65 to expel a preferred number of beads 40 from the tip 35. A tissue anchor 45 (FIG. 5) is provided at the tip 35 and is operably coupled to a knob 50 that is rotated for the extension and retraction of the tissue anchor 45. Tip 35 is placed adjacent to a selected surgical site and tissue anchor 45 is actuated to engage with wall tissue and anchor the tip 35 at the surgical site. De-actuation of the tissue anchor 45 releases the tissue anchor 45 from wall tissue. The tissue anchor 45 will be described in greater detail below.

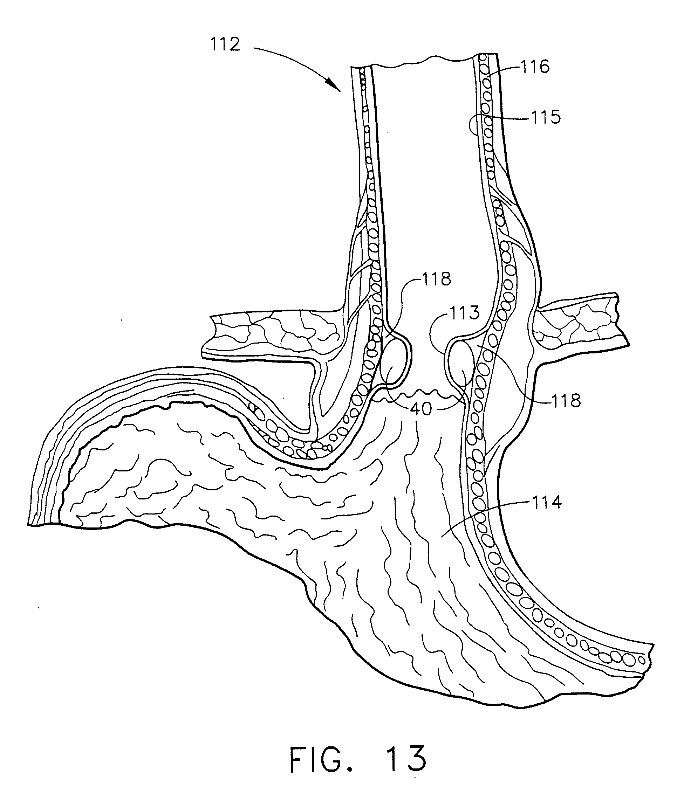

The surgical instrument 25 is adapted for use as a stand-alone instrument for bead 40 placement or used with other surgical instruments, such as the endoscope shown in FIG. 1. The endoscope 80 is commercially available and has a proximal endoscope handle 81 for the surgeon to grasp, a bendable or articulatable endoscope shaft 82 extending distally from the endoscope handle 81 for insertion into a patient, and a hollow operative channel 83 within the endoscope shaft 82. The operative channel 83 extends from an endoscope access port 85 to a distal end of the endoscope shaft 82 for the placement of surgical instruments within and flexible sheath 30 of the surgical instrument 25 is sized to slidably fit within the operative channel 83. The distal end of the endoscope shaft 82 has a viewing optics 84 located therein providing the surgeon with a view from the distal end of the endoscope 80. It is recommended that the surgical instrument 25 be placed within the endoscope 80 prior to the placement of the endoscope 80 into a patient 110 (FIG. 13).

FIG. 2 is an isometric view of the surgical instrument 25 wherein the elements within the elongated flexible sheath 30 are shown cross-sectioned and the elements of the handle 60 are shown exploded. The flexible sheath 30 is an elongated tubular structure having a flange 32 at a proximal end and the tip 35 at a distal end. A sheath passageway 31 extends between the tip 35 and the flange 32 and a feed member 55 moveably extends into the sheath passageway 31. The plurality of beads 40 is stored serially within the sheath passageway 31 adjacent to the tip 35. The elements and performance of the tip 35 will be described in greater detail below.

Beads 40 are elongated ovoids having a bead bore 41 extending therethrough. As shown, bead bore 41 extends through the bead 40 along the longest axis. The beads 40 of the present invention are between 3.1mm to 20mm in length along the longest axis and between 3.1mm to 8mm in diameter. Preferably, the beads 40 of the present invention are about 10mm in length and 4mm in diameter. Significantly, the size of the beads 40 greatly reduces the migration problem found with micro particles. Whereas the beads 40 for use with the surgical instrument and method of the present invention are ovoid in shape, and having a bead bore 41 therein, a wide variety of bead 40 sizes and shapes can be used and still meet the intent of the invention. Depending on the size of the beads 40 and the length of the surgical instrument 25, the number of beads 40 within the surgical instrument 25 can range from one to one thousand. Beads 40 are formed from a moisture absorbing material that expands after placement within wall tissue. Ideally, the bead 40 is formed from a dehydrated material that hydrates and expands upon exposure to bodily fluids and the beads 40 are formed from a single material such as but not limited to dehydrated cross-linked polyvinylpyrrolidone (also known as PVP and or hydrogel). The bead 40 can be formed from other materials that are moisture absorbing and expand, such as but not limited to open celled foam, closed cell foam, and sponges. It is significant that the beads 40 are used as a bulking material and do not require a carrier medium. The hydrated bulking material (beads 40) also remain constant in volume over time, unlike prior art bulking materials that use a carrier medium that can be absorbed by the body. Additionally, the bulking material increases in volume after placement in tissue.

The flexible sheath 30 has a flange 32 to attach the flexible sheath 30 to the cylindrical handle 60. Flange 32 is rotatably attached to a left half 61 and a right half 62 of the handle 60 by a collar 75. A strain relief 33 extends distally from the flange 32 and has a diameter larger than the flexible sheath 30. When the surgical instrument 25 is fully inserted into a surgical instrument such as the endoscope 80 (FIG. 1), strain relief 33 contacts the access port 85 of the endoscope 80 and stops further ingress. A bearing 76 is provided proximal to the flange 76 to guide feed member 55.

Feed member 55 is a flexible tubular member that extends proximally into the passageway 31 of the flexible sheath 30 and is operably coupled to the plurality of beads 40 stored adjacent to the tip 35. A portion of the feed member 55 extends distally from the flexible sheath 30 and into the handle 60. A bore 56 extends proximally to distally within feed member 55 along the longitudinal axis. The portion of the feed member 55 extending into the handle 60 is operably coupled to the firing trigger 66 by the indexing mechanism 65 described above. A plurality of V-grooves 57 are formed into the portion of the feed member 55 that extends into the handle 60, and form a portion of the indexing mechanism 65. The plurality of V-grooves 57 are equally spaced on feed member 55, and in the preferred invention, are spaced apart the length of a single bead 40. This spacing ensures that when the indexing mechanism 65 is actuated, the feed member 55 is moved to expel one bead 40. Each V-groove 57 is formed from a vertical face 58 and an angled face 59. The vertical faces 58 are normal to the longitudinal axis of the feed member 55 and are on the distal side of the V-groove 57. The angled faces 59 are on the proximal side of the V-grooves 57 and are angled relative to the longitudinal axis of the feed member 55.

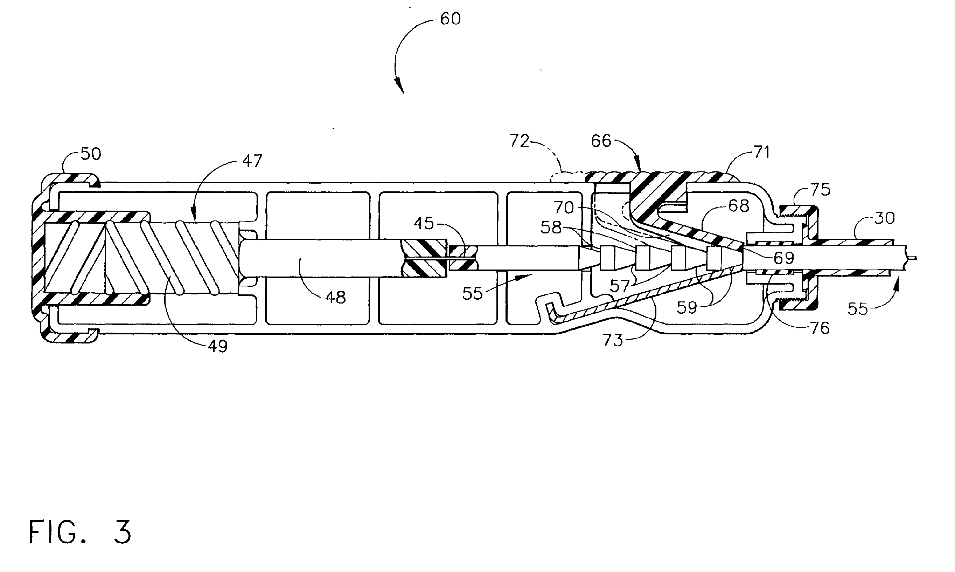

Indexing mechanism 65 also has the firing trigger 66 slidably mounted within a trigger slot 63 formed within the left half 61 and right half 62 of the handle 60. Firing trigger 66 has a finger pad 67 on the exterior of the handle 60 for the operator's finger, and proximal and distal motion of the firing trigger 66 actuates the indexing mechanism 65 for the expulsion of one bead 40 from the surgical instrument 25. A flexible drive blade 68 of "U" shaped cross section extends downwardly into the handle 60 to straddle and operably couple with the V-grooves 57 of the feed member 55 (FIG. 3). The "U" shaped flexible drive blade 68 has a channel 70 therein, and a vertical blade face 69 at a distal end. The vertical blade face 69 contacts the vertical surface 58 of one of V-grooves 57, and the channel 70 both straddles and contacts one of the angled faces 59 of the feed member 55. Operation of the indexing mechanism 65 will be described in greater detail below.

Indexing mechanism 65 also has a detent blade 73 that prevents proximal motion of the feed member 55. Detent blade 73 is an elongated cantilever member formed from a springy material such as spring steel or stainless steel. A proximal end of detent blade 73 is fixably mounted within the handle 60 and a distal end of the detent blade 73 engages with one of the V-grooves 57 of the feed member 55 (FIG.3). Proximal movement of the feed member 55 brings one of the vertical faces 58 of the V- grooves 57 into contact with the distal end of the detent blade 73 and prevents proximal motion of the feed member 55. Conversely, distal movement of feed member 55 brings one of the angled faces 59 into contact with the distal end of the detent blade 73 and deflects the detent blade 73 downwardly, allowing proximal motion of feed member 55.

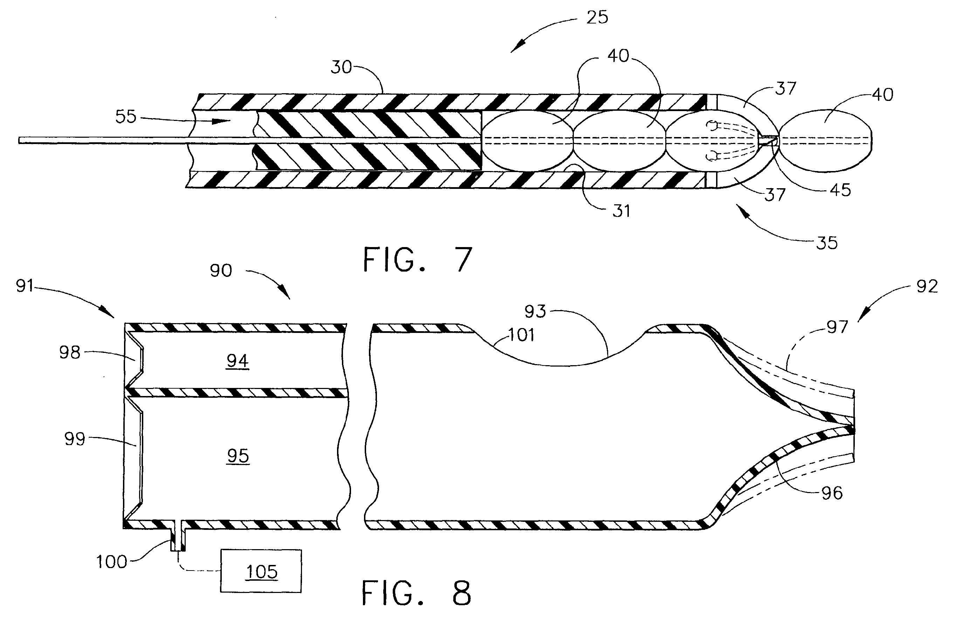

As described above, tissue anchor 45 attaches the tip 35 of the flexible sheath 30 to wall tissue at a selected surgical site prior to the placement of at least one bead 40 therein. Tissue anchor 45 is de-actuated to disengage the tissue anchor 45 from wall tissue. Tissue anchor 45 is generally an elongated flexible needle slidably mounted within the surgical instrument 25 having a distal point 46. Tissue anchor 45 extends distally from the tip 35 of the flexible sheath 30 and into the handle 60. Within the flexible sheath 30 the tissue anchor 45 extends through the bead bores 41 of the beads 40, and through the bore 56 of the feed member 55. The proximal portion of the tissue anchor 45 is fixedly attached to an elongated drive member 47. Drive member 47 has a distal guide portion 48 of rectangular cross section and a proximal threaded portion 49. The threaded portion 49 is operably engaged with the knob 50 and the knob 50 rotatably attached to the handle 60. Counter-clockwise rotation of the knob 50 engages the threads of threaded portion 49 and extends the tissue anchor 45 from the tip 35 of the flexible sheath 30 (FIG. 5) and clockwise rotation of the knob 50 retracts extended tissue anchor 45 back into the tip 35 (FIG. 7). The guide portion 48 of the drive member 47 is rectangular in cross section and slidably mounts within rectangular guide slots 64 within the left half 61 and right half 62 of the handle 60 and prevents rotation of the drive member 47 as the knob 50 is rotated.

FIGS. 3-8 are cross-sectional views showing the assembled elements of the surgical instrument 25. The assembled surgical instrument 25 and the interaction of the assembled components will now be described. The reader is advised to review FIGS. 3- 4 for the assembly and operation of the handle 60, and FIGS 5-8 for the assembly and operation of a distal portion of the flexible sheath 30.

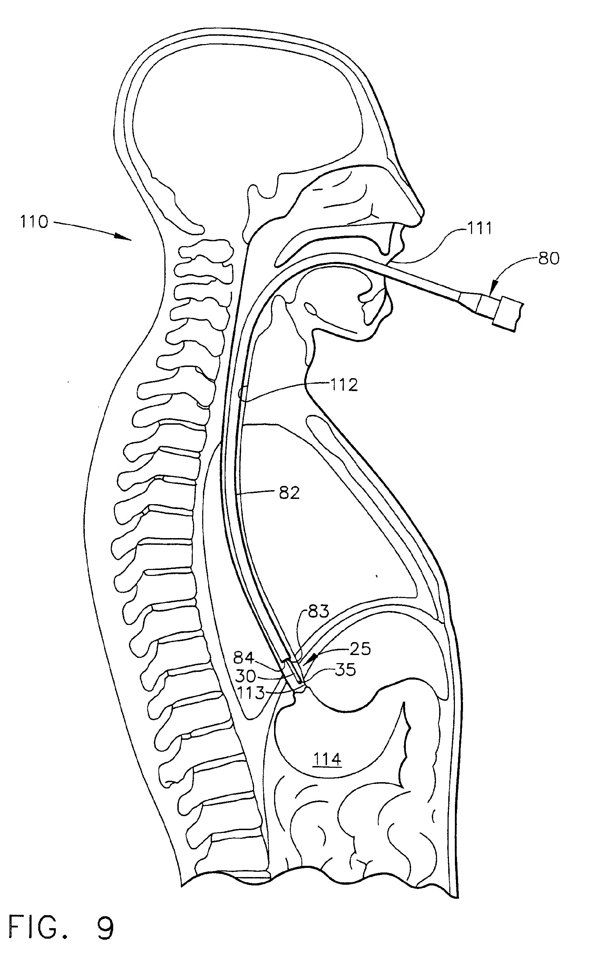

FIG. 3 shows the assembled handle 60 of the surgical instrument 25. The tissue anchor 45 has been moved distally from the retracted position (FIG. 8) to an extended position (FIG.5) by the counter clockwise rotation of knob 50. As shown in FIG. 5, tissue anchor 45 is extending beyond the tip 35. The amount that the tissue anchor 45 extends from the tip 35 is an amount between .005 inches and one inch. Preferably, the tissue anchor 45 is extended .12 inches from the tip 35. The reader should note that the tip 35 of the preferred invention is adapted to penetrate tissue and is tapered to facilitate penetration. The tip 35 of the preferred invention is also formed from at least two flexible petals 37 that are normally biased to the closed position of FIG. 5. It is also possible to form flexible petals 37 from a rigid material and to provide flexible hinges so that the flexible petals 37 clamshell open and closed.

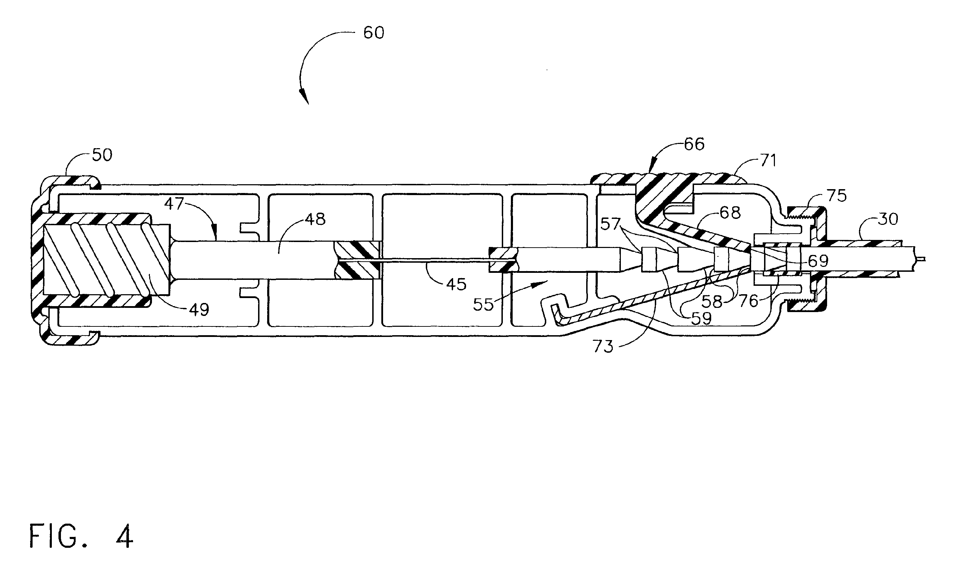

As shown in FIG. 3, the firing trigger 66 of the indexing mechanism 65 is shown in the initial distalmost position 71 prior to the actuation of the indexing mechanism 65. Firing trigger 66 is shown in the distalmost position 71 and will be reciprocated from the distalmost position 71, to the proximalmost position 72 (shown in dashed lines), and back to the distalmost position 71 to actuate the indexing mechanism 65. The length of the trigger slot 63 limits travel of the reciprocating firing trigger 66. Proximal movement of the firing trigger 66 to the proximal most 72 (dashed line) position slides the channel 70 of the drive blade 68 up and over the distalmost angled face 59 and into the next adjacent V-groove 57 (FIG. 4). Proximal motion of feed member 55 is prevented by the engagement of detent blade 73 with the distalmost vertical face 58 of the V-groove 57.

Distal movement of the firing trigger 66 from the proximal most position (dashed lines) indexes the feed member 55 distally (not shown) to eject a bead from the tip 35 (FIGS. 5-7). As the firing member 66 moves from the proximal most position (dashed lines) the vertical blade face 69 of the firing trigger 66 engages with the vertical faces 58 of the feed member 55 and moves the feed member 55 distally (not shown). As the feed member 55 moves distally, detent blade 73 is deflected downwards (not shown) by sliding contact with the distally moving angled face 59 until it passes and snaps into the next V-groove 57 as shown in FIG. 4.

FIGS. 6 and 7 shows the expulsion of a bead from the tip 35 of the flexible sheath 30. In FIG. 6, the extended tissue anchor 45 of FIG. 5 is retracted back into the tip 35 after the placement of the tip 35 into tissue and this will be described in detail later. The retraction of the extended tissue anchor 45 is accomplished by the clockwise rotation of the knob 50 to move the drive member 47 and the tissue anchor 45 proximally from the extended position of FIG. 3 to the retracted position of FIG. 4. In FIGS 5-7, the feed member 55 is moving distally, from the initial position of FIG 5, to the intermediate position of FIG. 6, and to the final position of FIG. 7. In these FIGS., feed member 55 pushes the distalmost bead 40 from an initial position of FIG. 5, into the flexible petals 37 (FIG. 6), and out of the distal tip 35 (FIG. 7). As the ovoid distalmost bead 40 is pushed into the flexible petals 37, the flexible petals 37 expand, creating an opening 38 for the passage and expulsion of the bead 40 from the tip 35. As the bead 40 passes through the flexible petals 37, the flexible petals 37 follow the profile of the bead 40, and expand and contract as the bead 40 is expelled. The flexible petals 37 return to the normal fully closed position of FIG. 7 after the expulsion of the bead 40.

In FIG. 7, distalmost bead 40 has been expelled from the tip 35, and the feed member 55 has stopped moving. The flexible petals 37 have returned to their normal fully closed position after the expulsion of the distalmost bead 40 and the closed flexible petals 37 restrain the next bead 40 within the sheath passageway 31.

FIG. 8 is an isometric view of an overtube 90 adapted for use in combination with the surgical instrument 25 of the present invention. The overtube 90 is placed within a lumen of a patient, such as the esophagus for the placement of a plurality of beads 40 within tissue of a patient. The overtube 90 is an elongated tubular structure formed from a flexible material having a proximal tube end 91 and a distal tapered end 92. A tissue receiving port 93 is located adjacent to the distal tapered end 92 and is provided for the reception of tissue therein. A first overtube passageway 94 extends from the proximal tube end and communicates with the tissue receiving port 93. The first overtube passageway 94 is sized for the reception of the flexible sheath 30 of the surgical instrument 25 therein. A second overtube passageway 95 is provided parallel to the first overtube passageway 94 and extending from the proximal tube end 91 to the tissue receiving port 93. The second overtube passageway 95 is provided for the passage of a second surgical instrument, such as a viewing endoscope 125, therein. A valve member 96 is provided within the distal tapered end 92 and the valve member 96 is normally closed. The valve member 96 is moveable from the closed position to an open position 97 shown in dashed lines by the passage of a surgical instrument such as the viewing endoscope 125 therethrough (not shown). The valve member 96 is preferably a Heimlich valve. The passage of the viewing endoscope 125 through the valve member 96 enables the surgeon to view the insertion and placement of the overtube 90 within the esophagus. A vacuum source 105 is operably coupled to the tissue receiving port 93 and is used to draw tissue into the tissue receiving port 93. Tissue receiving port 93 has an edge 101 and the importance of this edge will be described later. Valve member 96 is closed by the application of vacuum and forms a vacuum seal. The vacuum source 105 is coupled to overtube 90 at a vacuum nipple 100 adjacent to the proximal tube end of the overtube 90. A first seal 98 and a second seal 99 are located at the proximal ends of the first and the second overtube passageways 94 and 95 respectively, and form a vacuum seal with the surgical instruments inserted therein. The method of use of this instrument will be described in greater detail below.

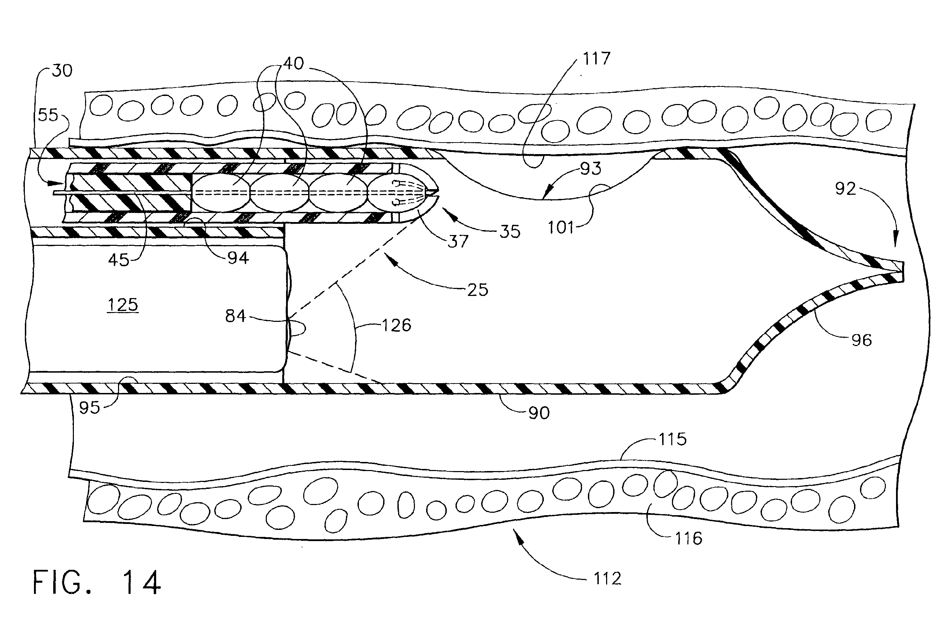

FIGS. 9-13, show a series of steps for using the above surgical instrument 25 for the placement of plurality of beads 40 within wall tissue of a lumen such as but not limited to an esophagus 112. The patient 110 is suffering from with Gastro-Esophageal Reflux Disease (GERD) and the beads 40 will be used to augment a lower esophageal sphincter 113 of the esophagus 112. The augmentation of the lower esophageal sphincter 113 reduces the migration of gastric secretions or reflux upwardly from a stomach 114 into the esophagus 112. The patient 110 is suffering from a severe case of GERD and a Nissen fundoplication surgery is deemed too radical based on the health of the patient 110. A minimally invasive surgery that provides minimal trauma and rapid recovery is indicated to repair the lower esophageal sphincter 113. The surgeon has elected to use the surgical instrument 25 of the present invention to augment the lower esophageal sphincter 113 with dehydrated beads 40 that swell when hydrated with bodily fluids (FIG. 14).

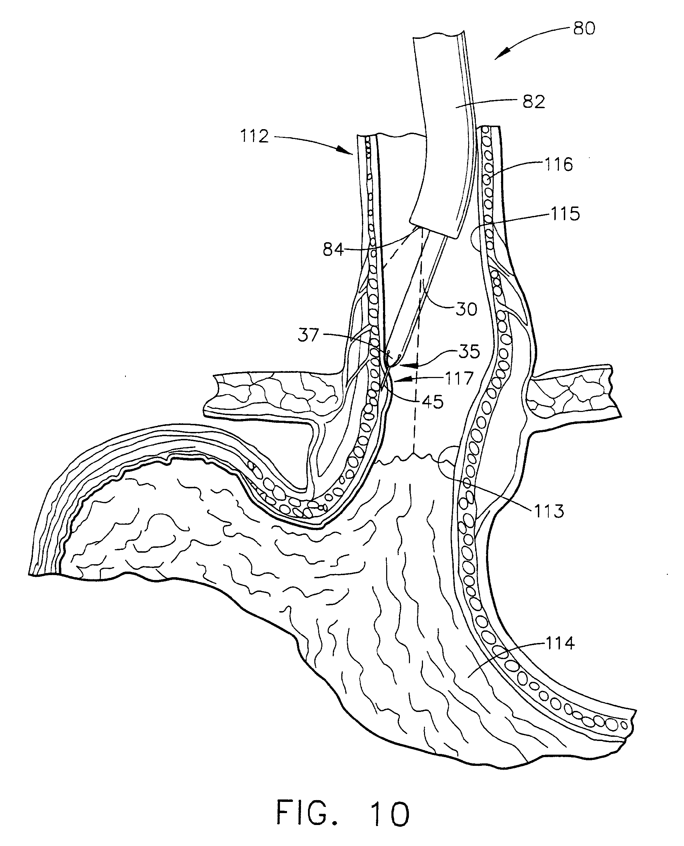

FIG. 9 is a section view of a patient 110 showing the placement of the surgical instrument 25 into the esophagus 112 of the patient 110. Surgical instrument 25 is preferably placed into the articulatable endoscope shaft 82 of endoscope 80 (FIG. 1) after placement of the endoscope 80 into a mouth 111 and esophagus 112 of the patient 110. A lower esophageal sphincter 113 of the patient 110 is viewed with the viewing optics 84 of the endoscope 80 to determine the patient's 110 condition. Once the lower esophageal sphincter 113 has been studied with the viewing optics 84, the surgical instrument 25 is placed into the operative channel 83 of the endoscope 80 (FIG.1) and into the esophagus 112. In FIG. 10, a lower portion of the flexible sheath 30 and the tip 35 of the surgical instrument 25 are shown protruding from the operative channel 83 of the endoscope 80.

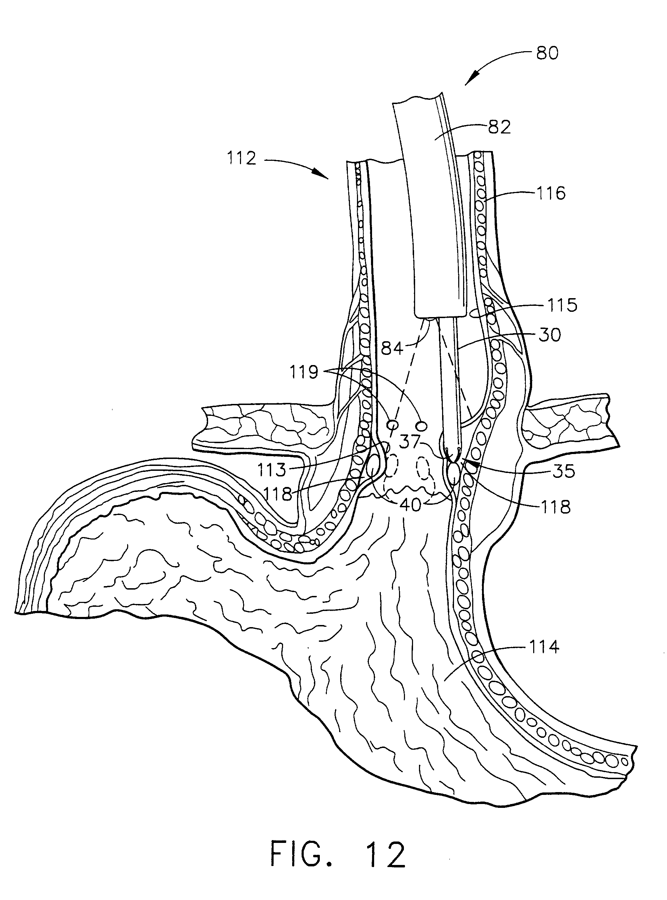

FIG. 10 shows the first step of attaching or anchoring the tip 35 of the surgical instrument 25 to wall tissue at a selected surgical site 117 at the lower esophageal sphincter 113. It is the object of the surgeon to augment the tissue adjacent to the lower esophageal sphincter 113 by placing a plurality of dehydrated beads 40 within wall tissue of the esophagus 112. More particularly, the beads 40 will be placed between an inner lining 115 and a surrounding muscle layer 116 of the esophagus 112. The lumen or esophagus 112 is a tubular structure and has a circumference. It is an object of the surgeon to place the beads 40 around at least a portion of the circumference during the augmentation of the lower esophageal sphincter 113 (FIG 12). To accomplish this, the surgeon first views the surrounding tissue with the viewing optics 84 and articulates the endoscope shaft 82 to place the tip 35 of the surgical instrument against the lower esophageal sphincter 113 at the desired surgical site 117. The tip 35 is deliberately placed at an acute angle with an upper portion of the esophagus 112 (FIG. 10) to facilitate the placement of the beads 40 between the inner lining 115 and surrounding muscle layer 116. Next, the tissue anchor 45 is engaged with the tissue wall of the esophagus 112 at the selected surgical site 117 by extending the tissue anchor 45 (FIG. 10) through the mucosal inner lining 115 and into the space between the inner lining 115 and the surrounding muscle layer 116. This action restrains the tip 35 relative to the selected surgical site 117.

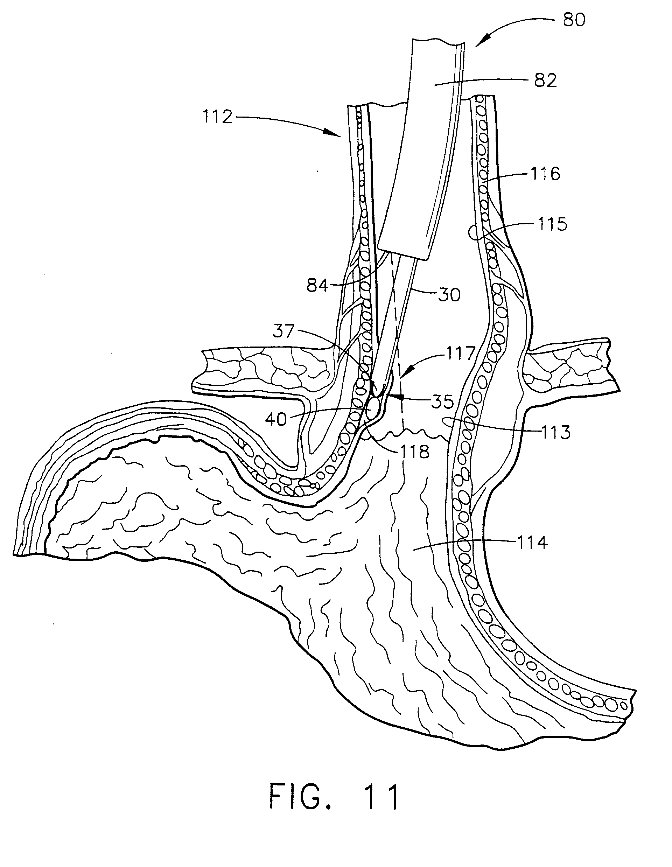

FIG. 11 shows the next step of augmenting the lower esophageal sphincter 113. In FIG. 12, the surgeon penetrates the mucosal inner lining 115 with the tip 35 of the surgical instrument 25 and an auguring motion. Once the tip 35 is in position between the mucosal inner lining 115 and the surrounding muscle layer 116, the tissue anchor 45 is retracted into the tip 35 and bead 40 is ejected from the surgical instrument 25. As shown in FIG. 6, the passage of the bead 40 through the flexible petals 37 deflects the flexible petals 37 outwardly. The deflection of the flexible petals 37, when positioned between the mucosal inner lining 115 and the surrounding muscle layer 116, locally dissects the mucosal inner lining 115 away from the surrounding muscle layer 116 and creates a tissue pocket 118 for the reception of the bead 40 therein. The bead 40 is then expelled from the tip 35 and into the tissue pocket 118 as shown. The tip 35 is then withdrawn from the selected surgical site 117.

FIG. 12 shows the placement of another of the plurality of beads 40 in the manner described above. The bead is placed within a surgical site 117 directly opposed to the surgical site 117 of FIG. 11. A number of penetration openings 119 are shown within the inner lining 115, each penetration opening 119 marks a surgical site 117 containing at least one of the dehydrated beads 40 to augment the lower esophageal sphincter. FIG. 13 shows the augmentation of the lower esophageal sphincter 113 by the beads 40 at approximately one week after the surgery. The dehydrated beads 40 have been hydrated and swelled by contact with bodily fluids and the penetration openings 119 (FIG. 12) within the inner lining 115 have healed.

FIGS. 14-17 teach a series of steps wherein the surgical instrument 25 is used in combination with the above described overtube 90 in an alternate method of treating the disease condition of the patient 110 (see above). That is, it is the goal of the surgeon to augment the lower esophageal sphincter 113 with the placement of a plurality of beads 40 around at least a portion of the circumference during the augmentation of the lower esophageal sphincter 113. Whereas the goal is the same as the previous method described above, the steps of the methods are different.

Each of the FIGS. 14-17 shows a cross-sectional view of a distal portion of overtube 90 placed within a lumen or esophagus 112. Overtube 90 is operably coupled to a vacuum source 105 (FIG. 8) and this is not shown in FIGS. 14-17. The purpose of this connection will be described later. The surgical instrument 25 is shown moveably placed within the first overtube passageway 94 and a viewing instrument or viewing endoscope 125 is slidably placed within the second overtube passageway 95 of the overtube 90. Viewing endoscope 125 is generally similar to the endoscope 80 shown in FIG. 1, but has a smaller endoscope shaft 82 than that of endoscope 80 of FIG. 1.

The viewing endoscope 125 has two uses when used with the overtube 90. First, the viewing endoscope 125 is used to view the inner wall of the esophagus 112 so that the tissue receiving port 93 can be positioned adjacent to a selected surgical site 117. Second, the viewing endoscope 125 is used to view the surgical instrument 25 as it places a plurality of beads 40 into wall tissue. To roughly position the overtube 90 within the esophagus 112 the endoscope shaft 82 of viewing endoscope 125 is moved distally within the first overtube passageway 94. The distal movement is continued until the distal end of the endoscope shaft 82 contacts and passes through the valve member 96 of the distal tapered end 92 of the overtube 90 (not shown). The passage of the distal end of the endoscope shaft 82 through the valve member 96 moves the valve member 96 to the open position 97 (FIG. 8) and extends the viewing optics 84 beyond the distal tapered end 92 of the overtube instrument. With the viewing optics 84 extended, the surgeon is able to view the inner wall of the esophagus 112 and to roughly position the overtube 90 near to the selected surgical site 117 (not shown). Fine positioning is accomplished by withdrawing the distal end of the endoscope shaft 82 back into the overtube 90 to the location shown in FIGS. 14-17 and using the view through the tissue receiving port 93 for fine positioning.

FIG. 14 shows the step of the placement of the overtube 90 into the esophagus 112 of the patient 110 adjacent to the lower esophageal sphincter 113. The placement of overtube 90 has been accomplished and the distal end of the endoscope shaft 82 is shown withdrawn into the overtube 90 to view the placement of beads 40 into wall tissue. It is desired to place the beads 40 between the inner lining 115 and the surrounding muscular tissue 116 of the esophagus 112 at the surgical site 117. Like the previous method described above, the beads 40 are to be placed around at least a portion of the circumference of the esophagus 112. Tissue receiving port 93 of overtube 90 is shown positioned adjacent to the selected surgical site 117 (FIG. 14).

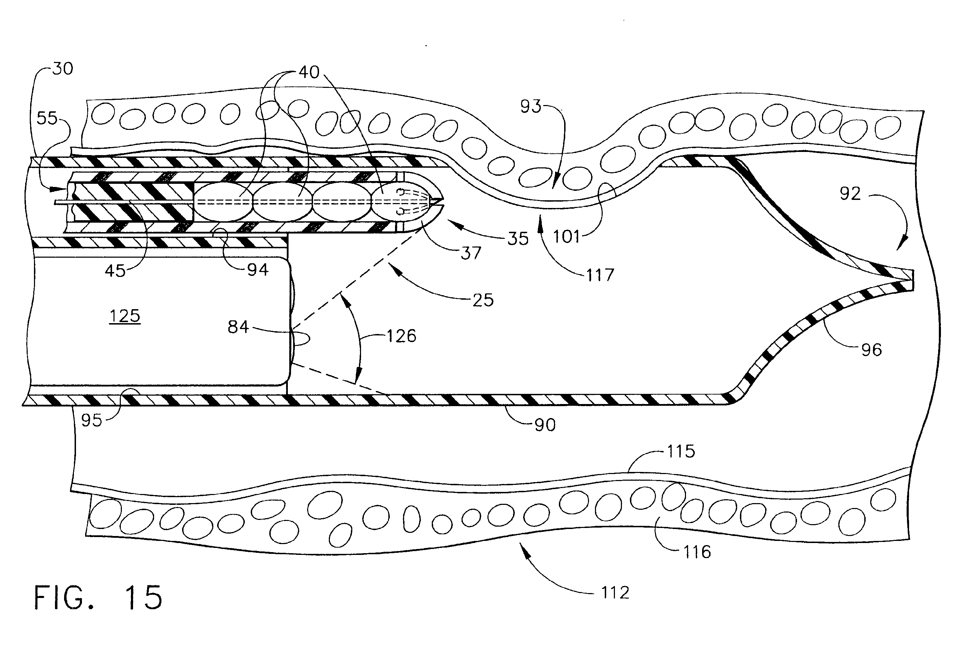

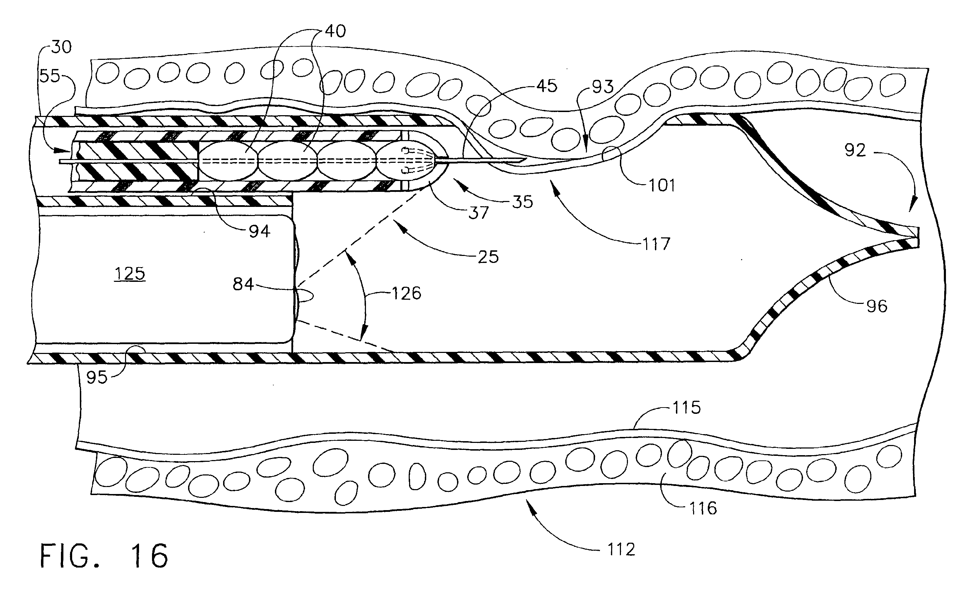

In FIG. 15, a portion of the tissue wall at the selected surgical site 117 is received within the tissue receiving port 93. The portion of the tissue wall at the selected surgical site 117 is drawn into the tissue receiving port 93 by the activation of vacuum source 105. Edge 101 of the tissue receiving port 93 is shaped to position the drawn portion of the tissue wall relative to the tissue anchor 45 and the tip 35 as shown. Turning now to FIG. 16, tissue anchor 45 is extended to penetrate the inner lining 15 at the apex of the drawn portion of the tissue wall, at the selected surgical site 117. Thus, the edge 101 of the overtube 90 aligns the tissue wall at the selected surgical site 117 relative to the tissue anchor 45 and the tip 35 of surgical instrument 25.

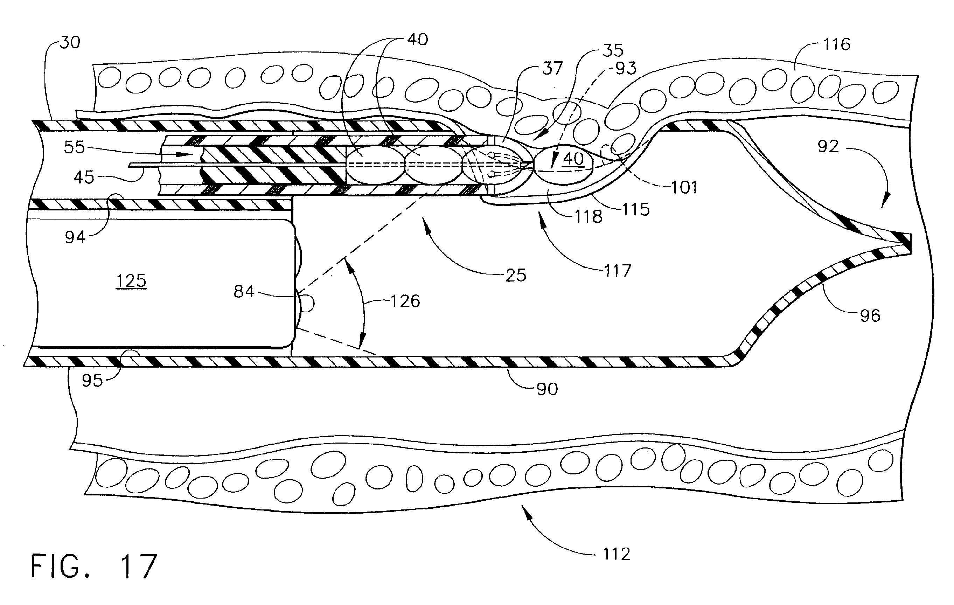

FIG. 17 shows the tip 35 of the surgical instrument 25 within the tissue wall at the selected surgical site 117, after the ejection of a bead therein. Prior to the view of FIG. 17, the tip 35 of the surgical instrument penetrates between the inner lining 115 and the surrounding muscle layer 116 to the position shown in FIG. 17. After the tip 35 has penetrated the inner lining 115, the tissue anchor 45 is retracted into the tip 35 of the surgical instrument 25 from the position of FIG. 16 to the position of FIG. 17. After retracting the tissue anchor 45, the distalmost bead 40 is ejected from the tip 35 (not shown) to create the tissue pocket 118 for the bead 40 (FIG. 17).

After placement of the bead 40, the vacuum source 105 is turned off and the surgical site 117 containing the placed bead 40 is released from the tissue receiving port 93 (not shown). The placement of beads 40 around the circumference of the lower esophageal sphincter 113 continues until the augmentation is complete similar to that shown in FIG 12. The surgery is completed when overtube 90 is withdrawn from the patient 110 (not shown).

While preferred embodiments of the present invention have been shown and described herein, it will be obvious to those skilled in the art that such embodiments are provided by way of example only. Numerous variations, changes, and substitutions will now occur to those skilled in the art without departing from the invention. Accordingly, it is intended that the invention be limited only by the spirit and scope of the appended claims.

## Claims

1. A surgical instrument for the placement of a plurality of beads within a tissue wall surrounding a lumen in a patient, the plurality of beads creating or augmenting a sphincter in the tissue wall, said surgical instrument comprising:
an elongated flexible sheath having proximal and distal ends and a sheath passageway extending therebetween, said sheath passageway having the plurality of beads stored therein;
a tip at said distal end of said elongated flexible sheath, said tip being adapted for penetrating tissue and said tip having at least one opening therein for the explusion of at least one of the plurality of beads therefrom;
a feeding member extending into said sheath passageway of said elongated flexible sheath and operably coupled to the plurality of beads, said feeding member being moveable in said sheath passageway for the explusion of at least one of the plurality of beads from said tip; and
a tissue anchor at said tip of said elongated flexible sheath, said tissue anchor being actuatable to engage with the tissue wall, said tissue anchor restraining said tip relative to a selected site for the placement of at least one of the plurality of beads therein, and said tissue anchor being deactuatable to disengage said tissue anchor from the tissue wall.

2. The surgical instrument of claim 1 wherein said tissue anchor is a needle, and when said tissue anchor is actuated, said needle moves from a retracted position within said tip to an extended position within the tissue wall.

3. A surgical instrument for the placement of a plurality of beads within a tissue wall surrounding a lumen in a patient, said beads creating or augmenting a sphincter in the tissue wall, said surgical instrument comprising:
an elongated flexible overtube having a proximal end and a distal tapered end, said elongated flexible overtube having at least one tissue receiving port for the reception of a tissue portion therein, said at least one tissue receiving port being adjacent to said tapered end and communicating with a first overtube passageway extending from said tissue receiving port to said proximal end of said elongated flexible overtube;
an elongated flexible sheath movably located within said first overtube passageway, said elongated flexible sheath having proximal and distal ends and a sheath passageway extending therebetween, said sheath passageway having a plurality of beads stored therein;
a tip at said distal end of said elongated flexible sheath, said tip being for penetrating the tissue portion drawn within said tissue receiving port of said elongated flexible overtube, said tip having at least one opening therein for the expulsion of at least one of the plurality of beads therefrom;
a feeding member extending into said sheath passageway of said elongated flexible sheath and operably coupled to said plurality of beads, said feeding member being moveable in said sheath passageway for the expulsion of at least one of the plurality of beads from said tip of said flexible sheath; and
a tissue anchor at said tip of said elongated flexible sheath said tissue anchor being actuatable to engage with the tissue portion within said tissue receiving port of said elongated flexible overtube, said tissue anchor restraining said tip relative to the tissue portion for the placement of at least one of the plurality of beads therein, and said tissue anchor being de-actuatable to disengage said tissue anchor from the tissue wall.

4. The surgical instrument of claim 3 further comprising a vacuum source operably communicating with said tissue receiving port, said vacuum source being for drawing the tissue portion therein.

5. The surgical instrument of claim 4 further comprising a valve member within said tapered end of said elongated flexible overtube, said valve member extending therethrough and communicating with said tissue receiving port, said valve member being moveable from a normally closed position to an open position.

6. The surgical instrument of any one of claims 3 to 5 further comprising a second overtube passageway within said elongated flexible overtube, said second overtube passageway being generally parallel to said first overtube passageway and extending from said tissue receiving port to said proximal end of said elongated flexible overtube, said second overtube passageway being for the reception of a surgical instrument therein.

7. The surgical instrument of any one of claims 1 to 6 wherein said tip is tapered for penetrating tissue.

8. The surgical instrument of claim? wherein said tip has at least two flexible petals, said flexible petals deflecting from a closed position for penetrating the tissue wall to an expanded position to create an opening for the passage and explusion of at least one of the plurality of beads therefrom and when said flexible petals are in the expanded position said expanded flexible petals create a tissue pocket in the tissue wall for the reception of at least one of the plurality of beads therein.

9. The surgical instrument of claim 8 wherein the flexible petals of said tapered tip are deflected outwardly by contact with at least one bead as the at least one bead passes through said flexible petals.

10. The surgical instrument of any one of claims 1 to 9 further comprising an indexing mechanism operably coupled to said feeding member, wherein when said indexing mechanism is actuated, a preferred number of said plurality of beads are ejected from said surgical instrument into the tissue wall.
